Europäisches Patentamt

European Patent Office

Office européen des brevets

(11) Numéro de publication: **0 496 671 B1**

# (12) FASCICULE DE BREVET EUROPEEN

(45) Date de publication du fascicule du brevet: **08.11.95** (51) Int. Cl.6: **D21C 5/00**, C12N 9/08

(21) Numéro de dépôt: **92400161.3**

(22) Date de dépôt: **22.01.92**

(54) **Traitement enzymatique d'une pâte ligno-cellulosique chimique.**

(30) Priorité: **25.01.91 FR 9100870**

(43) Date de publication de la demande:
**29.07.92 Bulletin 92/31**

(45) Mention de la délivrance du brevet:
**08.11.95 Bulletin 95/45**

(84) Etats contractants désignés:
**AT BE CH DE DK ES FR GB GR IT LI LU NL SE**

(56) Documents cités:
**EP-A- 0 244 262**
**EP-A- 0 371 712**

**ABSTRACT BULLETIN OF THE INSTITUTE OF PAPER CHEMISTRY. vol. 58, no. 8, Février 1988, APPLETON US, p. 1038;RAMACHAN-DRA, M; CRAWFORD, D.L.; POMETTO, A.L.:"Extracellular enzyme activities during lignocellulose degradation by Streptomyces spp.: comparative study of wild-type and genetically manipulated strains"**

(73) Titulaire: **SMURFIT-CELLULOSE DU PIN**
**Usine de Facture**
**Allée des Fougères**
**F-33380 Biganos (FR)**

(72) Inventeur: **Arbeloa, Marguerite**
**19 Bis Chemin Lalaurie**
**F-33140 Villenave d'Ornon (FR)**
Inventeur: **de Leseleuc, Joel**
**2 Allée Joliot-Curie**
**F-33700 Merignac (FR)**
Inventeur: **Goma, Gérard**
**30 Rue de Salas**
**F-31520 Ramonville-St-Agne (FR)**
Inventeur: **Pommier, Jean-Claude**
**35 Allée de Gascogne**
**F-33170 Gradignan (FR)**

(74) Mandataire: **Muller, René et al**
**SAINT-GOBAIN RECHERCHE**
**39, quai Lucien Lefranc-BP 135**
**F-93303 Aubervilliers Cédex (FR)**

Rank Xerox (UK) Business Services
(3.10/3.09/3.3.3)

**Description**

L'invention a trait à l'industrie papetière. Elle a pour objet un traitement enzymatique des pâtes ligno-cellulosiques chimiques.

En effet, ces pâtes sont obtenues à partir de bois, essentiellement constitué d'une matrice fibreuse faite de cellulose et d'hémicellulose, ainsi que de lignine, dont la structure macromoléculaire complexe est associée à ladite matrice.

Or la présence de lignine induit certains inconvénients : elle est en particulier sensible aux ultra-violets, provoquant la formation de produits brunâtres peu appréciés et néfastes à la blancheur généralement recherchée.

C'est pourquoi dans l'industrie papetière, on cherche à éliminer tout ou partie de la lignine. Cette élimination peut se faire par des traitements chimiques, la pâte obtenue étant alors une pâte chimique.

Lorsqu'en outre on veut obtenir du papier blanc, des traitements complémentaires, appelés traitements de blanchiment, s'avèrent nécessaires. Ceux-ci se déroulent généralement en plusieurs étapes, dont notamment des étapes de délignification à l'oxygène (étapes O), des étapes de chloration soit avec du chlore $Cl_2$ (étape C), soit avec du dioxyde de chlore $ClO_2$ (étape D), soit avec un mélange des deux (étapes C/D) où le taux de substitution de $Cl_2$ par $ClO_2$ peut atteindre de 50 à 100 %, ainsi que des étapes de réaction à l'hypochlorite (étapes H).

Peuvent intervenir également des étapes d'extraction alcaline (étapes E), éventuellement renforcée à l'oxygène (étapes E/O).

Ainsi, des procédés de blanchiment couramment utilisés mettent en oeuvre successivement les étapes suivantes :

C - E - H - D

ou C/D - E - D - E - D

ou O - C/D - E/O - D - E - D.

Mais ce type de procédé faisant intervenir des réactifs chlorés génère des sous-produits tels que des chlorolignines, peu biodégradables et pouvant être toxiques, qui, à certaines concentrations, sont susceptibles de polluer l'environnement lorsqu'ils sont rejetés sous forme d'effluents.

Des procédés alternatifs sont maintenant disponibles, utilisant par exemple l'ozone et/ou le peroxyde d'hydrogène, mais la blancheur des papiers qui en résultent peut encore être améliorée.

Le problème qui se posait étant donc, dans le cas de procédés de blanchiment avec des agents chlorés, d'obtenir une pâte au moins de même blancheur avec moins d'agents chlorés, et dans le cas de procédés de blanchiment ne faisant pas appel à des agents chlorés, d'améliorer la blancheur finale.

On a aussi proposé d'étudier l'utilisation de préparations enzymatiques dérivées de micro-organismes dans des traitements de séparation de la lignine de la pâte ligno-cellulosique. L'essentiel des études a été fait sur des enzymes fongiques :

Ainsi, les brevets US-A-4 690 895 et EP-A-345 715 décrivent tous deux une délignification des pâtes kraft par une préparation enzymatique dérivée du champignon

Phanerochaete chrysosporium.

Le brevet US-A-4 690 895 décrit un mutant dudit champignon, produisant des ligninases, en fait des peroxydases permettant de dégrader les composés aromatiques présents dans la lignine.

Le traitement envisagé nécessite une pâte en solution à pH acide compris entre 2 et 7 et préférentiellement de 4,5, enrichie en oxygène et alimentée en peroxyde d'hydrogène en présence de sulfate de manganèse : l'incubation dure 12 h à 39°C.

Il faut ensuite procéder à une seconde incubation avec de la soude, avant de décanter la pâte, et de la laver à l'eau.

Dans la demande de brevet EP-A-345 715 les enzymes décrites sont également des lignines-peroxydases et/ou des lignines-peroxydases associées à du manganèse de degré d'oxydation 2 (Mn II). Les conditions de traitement enzymatique sont similaires, la différence est la présence supplémentaire d'un détergent et d'un autre composé comme un acide a-hydroxylé. La manière préférentielle d'achever le traitement consiste en une extraction alcaline, un lavage à l'eau puis une extraction acide.

La demande de brevet EP-A-371 712, quant à elle, étudie le gène responsable de la formation de l'enzyme lignine-peroxydase dans la structure ADN de la bactérie Streptomyces viridosporus. Cette enzyme permet, selon la demande, d'oxyder la lignine en présence de peroxyde d'hydrogène à pH acide. EP-A-244 262 decrit le Streptomyces Cyaneus et la Thermonospora Mesophilia qu'on fait reagir sur un matériel ligno-cellulosique.

Selon une démarche un peu différente, la demande de brevet EP-A-373 107 propose un traitement par des hémicellulases, enzymes dérivées du champignon Aureobasidium pullulans. En dégradant les hémicelluloses, elles délogent la lignine qui y est associée de manière covalente, lignine ensuite séparée par extraction alcaline. Le traitement se fait également à pH acide, d'environ 5.

De même, les demandes de brevet EP-A-383 999 et EP-A-395 792 proposent également l'utilisation d'enzymes en général, et plus particulièrement d'hémicellulases, issues de bactéries de la famille de Streptomyces pour traiter des pâtes.

Ces dernières méthodes peuvent engendrer des inconvénients : en effet, les hémicellulases n'ont pas d'action directe spécifique sur la lignine, et ont tendance à dégrader non seulement les hémicelluloses liées de manière covalente à la lignine, et que l'on veut effectivement dégrader pour en libérer la lignine, mais aussi les hémicelluloses non liées à la lignine, ce qui n'est pas le but recherché : cette partie d'hémicellulose non liée à la lignine et pourtant solubilisée par ces enzymes peut causer une certaine chute de rendement d'une part. D'autre part, elle peut induire une augmentation de la demande biologique en oxygène (DBO) du milieu réactionnel, dont une partie se retrouve sous forme d'effluents dans l'environnement.

On constate donc que les enzymes d'origine fongique ou bactérienne connues pour leur action spécifique vis-à-vis de la lignine sont uniquement des peroxydases. Or traiter une pâte ligno-cellulosique avec des préparations à base de telles enzymes implique la présence obligatoire d'un co-substrat comme le peroxyde d'hydrogène, voire même la présence d'agents supplémentaires.

La seule alternative est d'utiliser des hémicellulases dont les inconvénients ont déjà été mentionnés.

En outre, on constate qu'avec la plupart des types de préparations enzymatiques cités ci-dessus, il est recommandé de se placer à un pH nettement acide. Or les pâtes en suspension aqueuse après avoir subi un traitement chimique alcalin au cours de la cuisson et/ou du blanchiment sont à un pH basique supérieur à 8. Cela implique donc que si l'on souhaite traiter une pâte après un tel traitement alcalin, il faut prévoir une étape préalable d'acidification.

L'invention présente a donc pour but un traitement enzymatique des pâtes ligno-cellulosiques chimiques, en vue d'en séparer la lignine, permettant d'obvier aux inconvénients des traitements enzymatiques précités, notamment en ayant une mise en oeuvre simplifiée, en particulier ne nécessitant ni acidification préalable ni co-substrat.

L'invention a pour objet un procédé de traitement d'une pâte ligno-cellulosique chimique en suspension aqueuse homogène par une préparation enzymatique dérivée de la bactérie Streptomyces viridosporus, et ayant une activité solubilisante de la lignine (ASL) en l'absence de co-substrat.

Par cette préparation enzymatique, on obtient bien une attaque spécifique sur la lignine. Cependant, la préparation enzymatique peut, en complément de cette activité, présenter une activité hémicellulolytique.

Le traitement concerne toutes les pâtes ligno-cellulosiques chimiques, et notamment les pâtes traitées au sulfate appelées pâtes Kraft.

Lors d'un traitement par des enzymes, le milieu réactionnel peut être plus ou moins approprié à l'action desdites enzymes. C'est pourquoi des conditions de pH et de température conviennent plus particulièrement pour obtenir une activité enzymatique optimale, et éviter également tout risque de dénaturation des enzymes.

Selon l'invention, le pH du milieu réactionnel est compris entre 7 et 9, et préférentiellement entre 8 et 8,5. Cette dernière fourchette correspond d'ailleurs au pH de croissance du milieu de culture du micro-organisme lui-même. Ce point est très avantageux lorsque ce traitement enzymatique est appliqué aux pâtes Kraft sortant du traitement chimique, car ces pâtes en solution sont alors déjà à un pH basique supérieur à 8. On peut donc traiter directement la pâte sans procéder à un ajustement à un pH acide. Le cas échéant, si cela s'avère nécessaire, on peut ajuster le pH de la pâte en solution pour avoir le pH optimum de 8 à 8,5, par ajout de soude ou d'acide notamment.

Le traitement s'effectue à une température généralement comprise entre 20 et 65°C, et préférentiellement entre 35 et 40°C, cette plage plus étroite donnant une activité enzymatique optimale correspond, comme pour le pH, à la plage de température du milieu de culture du micro-organisme, mais également approximativement à la plage de température rencontrée pour des pâtes Kraft sortant du traitement chimique.

L'action de la préparation enzymatique se poursuit selon l'invention pendant une durée généralement inférieure à 8 heures, ladite durée étant choisie en fonction de l'(les) activité(s) des différentes préparations enzymatiques dérivées de la bactérie Streptomyces viridosporus.

Le pourcentage en poids de la pâte à traiter par rapport à la solution aqueuse contenant la préparation enzymatique dans laquelle elle est en suspension peut varier de manière assez importante. Avantageusement, il sera compris entre 2 et 15 %.

L'activité solubilisante de la lignine ASL de la préparation enzymatique utilisée selon l'invention est préférentiellement comprise entre 0,001 et 0,1 unité par gramme de pâte (U/g).

L'activité hémicellulolytique, et plus particulièrement l'activité xylanasique de la préparation enzymatique utilisée est comprise préférentiellement entre 0 et 20 unité(s) par gramme de pâte.

Ce traitement enzymatique permet de séparer au moins une partie de la lignine contenue dans la pâte ligno-cellulosique. Les produits de la biodégradation spécifique de la lignine par les enzymes de Streptomyces viridosporus comprennent des polymères hydrosolubles à pH alcalin ou neutre, à degré de polymérisation moindre que la lignine elle-même. Il ne s'agit donc pas d'une biodégradation allant jusqu'à la minéralisation complète de la lignine. Mais le but de l'invention est bien atteint, puisque l'on peut séparer aisément les produits de dégradation de la pâte ligno-cellulosique, par simple essorage par exemple.

On constate, en outre, que, de manière surprenante, une telle préparation enzymatique peut avoir une activité solubilisante de la lignine importante, sans nécessiter la présence d'un quelconque co-substrat, en particulier tel que le peroxyde d'hydrogène, alors que jusque là les enzymes mises en évidence pour leur activité vis-à-vis de la lignine étaient essentiellement des peroxydases, ayant obligatoirement besoin au moins de peroxyde d'hydrogène pour agir. Le traitement selon l'invention est donc particulièrement avantageux, dans la mesure où, sans co-substrat ou autre agent, la mise en oeuvre dudit traitement est considérablement simplifiée et donc d'un coût moindre.

Le traitement selon l'invention peut être intégré à différents stades de la fabrication du papier. Il peut être intégré ou associé à un procédé habituel de blanchiment, soit en tant qu'étape préalable, soit en tant qu'étape intercalée entre une des séquences du procédé de blanchirent, soit en tant qu'étape postérieure audit procédé. Il peut être utilisé ou répété plusieurs fois.

Lorsque le traitement selon l'invention est une étape préalable au procédé de blanchiment, il peut être avantageux, selon le type de blanchiment prévu, de faire suivre le traitement enzymatique par un lavage à la soude, éventuellement suivi d'un lavage à l'eau.

Ce cycle traitement enzymatique - lavage alcalin - lavage à l'eau peut être répété plusieurs fois, notamment 2 à 3 fois, ce qui permet d'en augmenter l'efficacité.

Il est également envisageable d'utiliser la préparation enzymatique selon l'invention pour traiter les effluents issus d'une installation industrielle papetière.

Les résultats obtenus sont explicites, notamment en ce qui concerne le blanchiment, et seront détaillés ci-après. On observe dans tous les cas pour les pâtes ligno-cellulosiques chimiques quels que soient le procédé de blanchiment et l'origine du bois de départ (feuillus, résineux...), une augmentation sensible de la blancheur par rapport aux résultats obtenus pour une pâte n'ayant subi que le seul procédé de blanchiment habituel.

De plus, il est possible de réduire le taux de chlore actif mis en oeuvre par exemple à la première étape D d'un procédé de blanchiment type O-D-O-D-E-D d'au moins 25 % tout en obtenant une blancheur améliorée, rien qu'avec un seul traitement enzymatique préliminaire audit blanchiment.

Il est à noter également que, bien que le traitement permette d'augmenter la blancheur du papier obtenu, il n'en affecte en rien les propriétés mécaniques, alors que, de manière connue, essayer d'améliorer la blancheur finale au moyen d'étapes chimiques supplémentaires dans un procédé de blanchiment entraîne fréquemment une baisse des propriétés mécaniques du papier.

D'autres caractéristiques et avantages de l'invention apparaîtront au cours de la description détaillée ci-après d'exemples de réalisation.

Dans tous les exemples rapportés, les caractéristiques indiquées sont définies de la façon suivante :

- le Kappa (K) d'une pâte ligno-cellulosique permet d'évaluer le taux de lignine de la pâte. Sa détermination, bien connue dans le domaine papetier, est basée sur le principe de l'oxydation de la lignine de la pâte par le permanganate de potassium, et est donnée de manière exacte par la norme ISO-302. La connaissance du Kappa d'une pâte permet de définir le pourcentage massique de chlore actif par rapport à la pâte (noté Cl°) à utiliser lors de la première étape de chloration du procédé de blanchiment. La relation habituellement utilisée est la suivante : Cl° = 0,2 x K

- la blancheur est déterminée conformément à la norme ISO-2470. C'est la mesure du facteur de réflectance diffuse dans le bleu de la couche de papier étudiée, à l'aide d'un diffuseur parfait par réflexion.

- les activités des préparations enzymatiques dérivées de Streptomyces viridosporus sont déterminées de la manière suivante :

. l'activité solubilisante de la lignine des enzymes se détermine par mise en contact de la préparation enzymatique en suspension aqueuse avec de la ligno-cellulose sous forme de poudre désignée sous le terme PAB (poudre alcool benzène). Cette poudre est obtenue par extraction de la ligno-cellulose à partir de matériel végétal préalablement marqué spécifiquement sur la lignine

4

au carbone 14 d'après un protocole précisé dans un article de G. Alibert et A. Boudet (1979, Physiol-Veg. vol.17, pages 67-74). Cette mise en contact conduit à une solubilisation progressive de composés radioactifs. Il s'agit ensuite de doser lesdits composés. En pratique, la poudre de ligno-cellulose est mise en suspension dans un tampon Tris/HCl (0,3 M, pH 8,0 ou 8,5), à la concentration de 10 mg/ml.

Cette suspension est ajoutée à la solution enzymatique (à raison de 1 volume pour 2 volumes respectivement), avec agitation, à 37°C. Le suivi de la réaction se fait par le comptage de la radioactivité contenue dans le surnageant des prélèvements du milieu effectués au cours du temps. Les résultats sont exprimés en pourcentage de radioactivité solubilisée par rapport à la radioactivité initiale de la poudre de ligno-cellulose, ce pourcentage donnant directement le pourcentage de lignine dégradée solubilisée par rapport à la lignine initiale. On conviendra qu'une unité est définie comme 1 % de radioactivité solubilisé par minute.

. l'activité hémicellulolytique de la préparation enzymatique est fondée plus spécifiquement sur la présence de xylanases dans ladite préparation. Or les xylanases de Streptomyces viridosporus, en contact avec du xylane, libèrent dans le milieu réactionnel des sucres réducteurs, que l'on dose par la méthode de l'acide 3,5-dinitrosalicylique (DNS) de J.B. Summer et S.F. Howell (1935, J. Biol. Chem., vol 108, pages 51-54).

En pratique, une solution de xylane à 1 % (obtenue par mise en solution à température ambiante dans un tampon phosphate 0,1 M à pH 8) et la préparation enzymatique en solution sont mélangées volume à volume par agitation à 37°C ou à 50°C. La réaction est suivie par la mesure des sucres réducteurs dans les prélèvements du milieu. On peut ensuite, avec ces dosages, chiffrer l'activité xylanasique, en unité U selon la nomenclature internationale.

La souche de Streptomyces viridosporus utilisée ici est issue de celle déposée sous la référence ATCC 39115. De manière connue, on procède à la culture du micro-organisme en plusieurs stades, en erlenmeyer puis en fermenteur. De même, on sépare ensuite les bactéries, afin de recueillir le milieu extracellulaire contenant les enzymes produites par celles-ci. Lesdites enzymes font ensuite éventuellement l'objet de purification et/ou séparation.

Dans les essais décrits par la suite, la réaction enzymatique se fait sur une pâte ligno-cellulosique à 4 % massique en suspension aqueuse contenant la préparation enzymatique. Si nécessaire, le pH de la suspension est ajusté vers 8. La réaction se fait à 37°C sous agitation pendant un temps variable allant jusqu'à 8 heures. A la fin de la réaction, la pâte est essorée, et éventuellement lavée à la soude 1N puis à l'eau : lorsque l'on effectue ensuite sur la pâte un procédé de blanchiment dont la première séquence est une extraction alcaline à l'oxygène, on n'effectue en général pas ledit lavage à la soude.

Tous les pourcentages donnés sont des pourcentages massiques.

EXEMPLES 1 A 4 :

Les étapes de culture de Streptomyces viridosporus (S.v) sont résumées brièvement dans le tableau ci-dessous, dans les conditions habituelles de culture, c'est-à-dire à 37°C, avec régulation de pression d'oxygène et un pH compris entre 7,5 et 8.

| Etape | réacteur | | ensemencement | temps (h.) |
|-------|----------|------|---------------|------------|
| 1 | Erlen | 100 ml | 2 ml pellets stock | 24 |
| 2 | " | 1 l | S.v de l'étape 1, broyé | 12 |
| 3 | Braun | 15 l | S.v de l'étape 2 | 12 |

Puis on effectue une filtration du milieu de culture sur membranes de silicate-fibres de verre, pour éliminer les micro-organismes. On concentre ensuite le filtrat et on fait une première séparation des différentes enzymes par mise en contact des enzymes en solution avec un gel d'échange d'ions (C.M. sépharose), la solution étant tamponnée à pH 5 (citrate 20 mM). Les enzymes non liées au gel sont regroupées en une fraction désignée par le terme surnageant et récupérées par filtration et rinçage du gel. C'est ce surnageant qui va permettre de traiter trois types différents de pâtes ligno-cellulosiques.

L'activité solubilisante de la lignine de ce surnageant est de 0,5 Unité par litre de préparation enzymatique (U/l) et l'activité xylanasique de 100 U/l.

EXEMPLE 1

Le traitement enzymatique est effectué à pH 8, 37°C pendant 120 mn sur une pâte kraft à partir de bois de feuillus tels que le chêne, le hêtre, le chataignier, le peuplier ou un mélange de ceux-ci, qui subit ultérieurement un procédé de blanchiment dont les séquences sont les suivantes :
- une étape C de chloration : Cl° = 0,2 x Kappa
- une étape E d'extraction alcaline : NaOH = 2 %
- une étape H de réaction à l'hypochlorite : Cl° = 1,5 %
- une étape D de réaction au dioxyde de chlore : Cl° = 1 %

Ces résultats sont les suivants :

|  | Témoin | avec traitement enzymatique |
|---|---|---|
| Kappa après traitement | 12,8 | 11,3 |
| Blancheur en D | 87,4 | 88,1 |

EXEMPLE 2

Le même traitement dans les mêmes conditions est effectué sur une pâte kraft à partir de bois de résineux tels que l'épicéa et/ou le sapin qui subit ensuite un procédé de blanchiment dont les séquences sont les suivantes :
- une étape C/D, c'est à dire avec un mélange de chlore et de dioxyde de chlore : Cl° = 0,18 x Kappa (20 % $ClO_2$)
- une étape $E_1$ : NaOH = 2,5 %
- une étape $D_1$ : Cl° = 3 %
- une étape $E_2$ : NaOH = 1 %
- une étape $D_2$ : Cl° = 1,5 %

Les résultats sont les suivants :

|  | Témoin | avec traitement enzymatique |
|---|---|---|
| Kappa après traitement | 25,6 | 23 |
| Blancheur en $D_1$ | 66,4 | 71,5 |
| Blancheur en $D_2$ | 85 | 86,7 |

EXEMPLE 3

Le traitement enzymatique se déroule comme les précédents, cette fois également sur une pâte kraft de résineux, en l'occurence obtenue à partir de pins maritimes, dont le procédé de blanchiment ultérieur comporte les étapes suivantes :
- une étape $O_1$ d'attaque à l'oxygène en milieu alcalin : $P(O_2)$ = 3 bars, NaOH = 2,5 %
- une étape $D_1$ : Cl° = 0,2 x Kappa
- une étape $O_2$ : $P(O_2)$ = 3 bars, NaOH = 2 %
- une étape $D_2$ : Cl° = 2 %
- une étape E : NaOH = 1 %
- une étape $D_3$ : Cl° = 1 %

Les résultats sont les suivants :

|  | Témoin | avec traitement enzymatique |
|---|---|---|
| Kappa après traitement | 29,9 | 27,6 |
| Kappa après $O_1$ | 16,9 | 15,1 |
| Kappa après $O_2$ | 4,2 | 3,9 |
| Blancheur en $D_2$ | 78,6 | 79,7 |
| Blancheur en $D_3$ | 90,7 | 91,8 |

EXEMPLE 4

La nature de la pâte, celle du traitement enzymatique et celle du procédé de blanchiment sont quasiment les mêmes que celles données à l'exemple 3. La seule différence réside dans le fait qu'à l'étape $D_1$ de blanchiment, on a utilisé du chlore actif Cl° dans la quantité suivante : Cl° = 0,15 x K, c'est-à-dire que l'on a utilisé environ 25 % de moins de chlore actif pour cette étape.
Les résultats sont les suivants :

|  | Témoin | avec traitement enzymatique |
|---|---|---|
| Kappa après $O_2$ | 4,7 | 4,3 |
| Blancheur en $D_2$ | 76,5 | 77,7 |
| Blancheur en $D_3$ | 89,9 | 91,1 |

On constate donc que pour les trois types de pâte, l'amélioration de la blancheur est très sensible, de l'ordre de 1 à 2 % en blancheur après le blanchiment, et de l'ordre de 1 à 2 point(s) sur la valeur du kappa juste après le traitement enzymatique. Ce sont des améliorations tout à fait intéressantes, dans la mesure où il est plutôt ardu d'obtenir un gain de blancheur au delà d'une blancheur de l'ordre de 85 à 90 %.

Les exemples 3-4 montrent, en outre, qu'en utilisant 25 % de chlore actif en moins dans l'étape $D_1$ du procédé de blanchiment cité, on obtient grâce au traitement enzymatique une blancheur non seulement équivalente, mais supérieure, puisque on a en final une blancheur de 91,1 dans l'exemple 4 alors que la blancheur du témoin dans l'exemple 3 n'est que de 90,7.

Cela signifie que l'on peut choisir en fait, soit d'axer l'objectif sur une blancheur maximale en gardant le taux de chlore habituel utilisé dans le procédé de blanchiment, soit d'axer l'objectif vers une diminution très sensible dudit taux de chlore tout en maintenant une blancheur satisfaisante, ou tout autre objectif situé entre ces deux limites.

EXEMPLES 5 A 6

Les étapes de culture de la bactérie sont identiques. On effectue de même après la culture une filtration des bactéries, on concentre le filtrat sans effectuer de séparation analytique.
L'activité solubilisante de la lignine de la solution enzymatique est de 0,8 U/l.

EXEMPLE 5

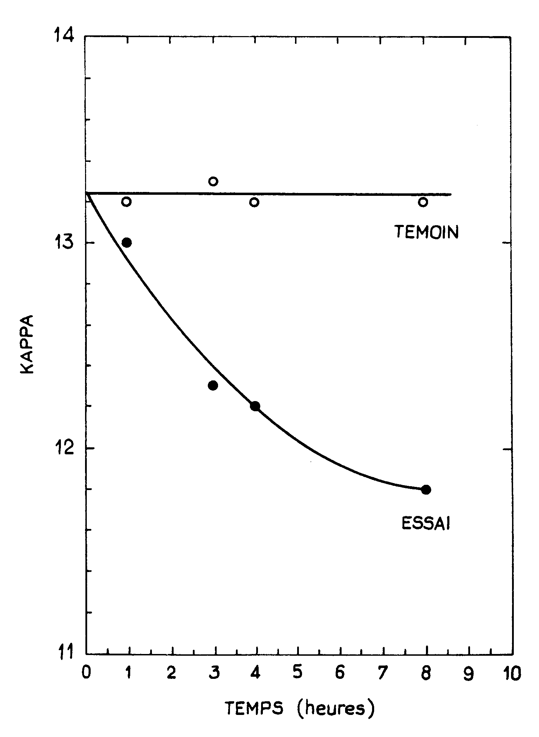

On traite une pâte à partir de bois de feuillus, venant de subir le traitement chimique par cette solution enzymatique à une température de 37° C et un pH de 8,7. On mesure en fonction du temps (0-8 heures) l'évolution du Kappa de la pâte, le Kappa de départ étant de l'ordre de 13,2 par rapport à un témoin. La courbe obtenue est donnée sur la planche unique ci-jointe. On constate une nette baisse du Kappa, qui passe à environ 11,9 au bout de 8 heures, soit un gain de 1,3 point.

EXEMPLE 6

On traite de la même façon une même pâte, mais cette fois pendant 4 heures. Ensuite, on lave la pâte à la soude 1N, puis à l'eau. Puis on effectue à nouveau le même cycle de traitement. L'évolution du Kappa en fonction du nombre de traitements est donnée dans le tableau ci-dessous.

```
traitement enzymatique
Kappa avant le 1er traitement  :  13,9
Kappa après le 1er traitement  :  12,1
Kappa après le 2ème traitement :  10,1
```

On constate que faire deux fois successivement le traitement enzymatique permet d'améliorer notablement la valeur de Kappa.

**Revendications**

1. Procédé de traitement d'une pâte ligno-cellulosique chimique par une préparation enzymatique, **caractérisé en qu'**il fait partie du procédé de blanchiment de la pâte en vue de fabriquer du papier et **en ce qu'**il consiste à faire agir directement sur ladite pâte en suspension aqueuse homogène, sans acidification préalable et pendant une durée inférieuré à 8 heures, une préparation contenant des enzymes dérivées de la bactérie Streptomyces Viridosporus et ayant une activité solubilisante de la lignine en l'absence de co-substrat.

2. Procédé de traitement selon la revendication 1, **caractérisé en ce que** ladite préparation contenant des enzymes présente également une activité hémicellulolytique.

3. Procédé de traitement suivant une des revendications 1 ou 2, **caractérisé en ce que** la pâte ligno-cellulosique chimique est une pâte Kraft.

4. Procédé de traitement selon une des revendications 1 à 3, **caractérisé en ce que** le milieu réactionnel a un pH compris entre 7 et 9 et de préférence entre 8 et 8,5.

5. Procédé de traitement selon une des revendications 1 à 4, **caractérisé en ce que** le milieu réactionnel est porté à une température comprise entre 20°C et 65°C et de préférence entre 35°C et 40°C.

6. Procédé de traitement selon l'une des revendications précédentes, **caractérisé en ce que** l'action de la préparation enzymatique est poursuivie pendant une durée de 15 minutes à 3 heures.

7. Procédé de traitement selon une des revendications précédentes, **caractérisé en ce que** la concentration en pourcentage massique de la pâte dans le milieu réactionnel est comprise 2 et 15% et de préférence égale à 4%.

8. Procédé de traitement selon une des revendications précédentes, **caractérisé en ce que** l'activité solubilisante de la lignine de la préparation contenant les enzymes est comprise entre 0,001 et 0,1 U par gramme de pâte traitée.

9. Procédé de traitement selon une des revendications 2 à 8, **caractérisé en ce que** l'activité hémicellulolytique de la préparation contenant les enzymes est une activité xylanasique inférieure ou égale à 20 U par gramme de pâte traitée.

10. Procédé de traitement selon une des revendications précédentes, **caractérisé en ce que** la bactérie Streptomyces Viridosporus est obtenue à partir de la souche référencée ATCC 39115.

**11.** Procédé de traitement enzymatiqmue selon une des revendications précédentes, **caractérisé en ce que** l'on effectue après l'action de la préparation enzymatique un lavage à la soude de la pâte, éventuellement suivi d'un lavage à l'eau.

**12.** Procédé **caractérisé en ce qu'**il consiste à effectuer plus d'une fois le traitement enzymatique selon une des revendications précédentes.

**Claims**

**1.** Process for the treatment of a chemical lignocellulose pulp by an enzymatic preparation, characterized in that it forms part of the pulp bleaching process for making paper and in that on said pulp in homogeneous aqueous suspension is made to act, without prior acidification and for a time shorter than 8 hours, a preparation containing enzymes having derived from the bacteria Streptomyces viridosporus and having a lignin solubilizing activity in the absence of the cosubstrate.

**2.** Treatment process according to claim 1, characterized in that the preparation containing enzymes also has a hemicellulolytic activity.

**3.** Treatment process according to either of the claims 1 and 2, characterized in that the chemical lignocellulose pulp is a kraft pulp.

**4.** Treatment process according to any one of the claims 1 to 3, characterized in that the reaction medium has a pH between 7 and 9 and preferably between 8 and 8.5.

**5.** Treatment process according to any one of the claims 1 to 4, characterized in that the reaction medium is heated to a temperature between 20 and 65°C and preferably between 35 and 40°C.

**6.** Treatment process according to any one of the preceding claims, characterized in that the action of the enzymatic preparation is continued for between 15 minutes and 3 hours.

**7.** Treatment process according to any one of the preceding claims, characterized in that the concentration in percent by weight of the pulp in the reaction medium is between 2 and 15 and is preferably 4%.

**8.** Treatment process according to any one of the preceding claims, characterized in that the lignin solubilizing activity of the preparation containing the enzymes is between 0.001 and 0.1 U/gramme of pulp treated.

**9.** Treatment process according to any one of the claims 2 to 8, characterized in that the hemicellulolytical activity of the preparation containing the enzymes is a xylanase activity equal to or below 20 U/gramme of treated pulp.

**10.** Treatment process according to any one of the preceding claims, characterized in that the bacterium Streptomyces viridosporus is obtained from the strain designated ATCC 39115.

**11.** Enzymatic treatment process according to any one of the preceding claims, characterized in that following the enzymatic preparation action, the pulp is washed with soda and optionally then with water.

**12.** Process, characterized in that it consists of performing the enzymatic treatment according to any one of the preceding claims.

**Patentansprüche**

**1.** Verfahren zur Behandlung eines Holzzellstoffbreis mit einer enzymatischen Zubereitung, **dadurch gekennzeichnet, daß** es Teil des Bleichverfahrens für den Brei für die Herstellung von Papier ist und daß es darin besteht, auf den in homogener wäßriger Suspension befindlichen Brei ohne vorheriges Ansäuern während einer Zeitdauer von weniger als 8 Stunden eine Zubereitung direkt einwirken zu lassen, die von der Bakterie Streptomyces Viridosporus abgeleitete Enzyme enthält und ohne Cosubstrat eine Ligninsolubilisierende Aktivität besitzt.

2. Behandlungsverfahren nach Anspruch 1, **dadurch gekennzeichnet, daß** die enzymhaltige Zubereitung auch eine Hemicellulose-Aktivität besitzt.

3. Behandlungsverfahren nach Anspruch 1 oder 2, **dadurch gekennzeichnet, daß** der Holzzellstoffbrei ein Kraft-Brei ist.

4. Behandlungsverfahren nach einem der Ansprüche 1 bis 3, **dadurch gekennzeichnet, daß** das Reaktionsmedium einen pH-Wert von 7 bis 9 und vorzugsweise zwischen 8 und 8,5 aufweist.

5. Behandlungsverfahren nach einem der Ansprüche 1 bis 4, **dadurch gekennzeichnet, daß** das Reaktionsmedium auf eine Temperatur von 20 bis 65 °C und vorzugsweise zwischen 35 und 40 °C gebracht wird.

6. Behandlungsverfahren nach einem der vorhergenden Ansprüche, **dadurch gekennzeichnet, daß** die Wirkung der enzymatischen Zubereitung während einer Zeitdauer von 15 Minuten bis 3 Stunden anhält.

7. Behandlungsverfahren nach einem der vorhergenden Ansprüche, **dadurch gekennzeichnet, daß** die Konzentration in Masseprozent des Breis im Reaktionsmedium 2 bis 15 % und vorzugsweise 4 % beträgt.

8. Behandlungsverfahren nach einem der vorhergenden Ansprüche, **dadurch gekennzeichnet, daß** die Ligninsolubilisierende Aktivität der enzymhaltigen Zubereitung 0,001 bis 0,1 U pro Gramm zu behandelnden Breis beträgt.

9. Behandlungsverfahren nach einem der Ansprüche 2 bis 8, **dadurch gekennzeichnet, daß** die Hemicellulose-Aktivität der enzymhaltigen Zubereitung eine Xylanase-Aktivität von kleiner oder gleich 20 U pro Gramm zu behandelnden Breis ist.

10. Behandlungsverfahren nach einem der vorhergenden Ansprüche, **dadurch gekennzeichnet, daß** die Bakterie Streptomyces Viridosporus aus dem Referenzstamm ATCC 39115 erhalten ist.

11. Verfahren der enzymatischen Behandlung nach einem der vorhergenden Ansprüche, **dadurch gekennzeichnet, daß** der Brei nach Einwirkung der enzymatischen Zubereitung mit Natronlauge und gegebenenfalls danach mit Wasser gewaschen wird.

12. Verfahren, **dadurch gekennzeichnet, daß** es in der mehrmaligen Durchführung der enzymatischen Behandlung nach einem der vorhergehenden Ansprüche besteht.

10